# EUROPEAN PATENT APPLICATION

(11) **EP 2 446 927 A1**
(43) Date of publication of application: **02.05.2012**
(21) Application number: 10189246.1
(22) Date of filing: 28.10.2010
(51) Int. Cl.: A61N 1/39

(54) **Ultra-short high voltage electric defibrillation pulses**

(71) Applicant: Schiller Medical S.A.S., 67162 Wissembourg Cedex (FR)
(72) Inventor: Schiller, Alfred, 8914 Aeugst a. A. (CH); Schmid, Johann-Jakob, 8911 Rifferswil (CH); Didon, Jean-Philippe, 67250 Merkwiller-Pechelbronn (FR)
(74) Representative: Hepp Wenger Ryffel AG

(57) **Abstract**

An external defibrillator for delivering a defibrillation shock to the heart of a patient under cardiac arrest is provided. The defibrillator comprises an energy source for providing electrical energy, at least one capacitor for building up and storing an electrical charge for delivery to the patient and at least one set of electrodes to be applied externally to the patient's skin. The external defibrillator is adapted to provide at least one pulse train comprising at least one individual pulse of a duration of from 0.4 to 3 ms. Waveforms and methods of operating an automated external defibrillator are further provided.

## Description

The present invention relates to defibrillators, more particularly to portable defibrillators and automated external defibrillators, as well as to methods for operating a defibrillator, according to the preambles of the independent claims.

Out-of-hospital defibrillation therapy has become the standard of care of first-responding for patients suffering from cardiac arrest due to ventricular fibrillation. The ratio of public spaces equipped with defibrillators has been steadily increasing. Furthermore, the design of automated external defibrillators, which can be used by first-responders with minimal or no training, has further supported the proliferation of defibrillators. These devices are usually equipped with advanced recognition and treatment protocols for patients undergoing an event of cardiac arrest.

Cardiac distress is characterized by a magnitude of symptoms, from acute chest pain up to loss of consciousness with or without pulse. The delay from the onset of symptoms up to a potentially life threatening event of cardiac distress can be very short. Therefore, the availability of a defibrillator is of crucial importance for increasing the survivability of patients under cardiac arrest. For that end, sufficient devices must be installed and easily locatable by the first-responders.

Still, a majority of life threatening events of cardiac arrest happen in a private environment, such as at home. A major hindrance of equipping households with defibrillators is the comparatively large price, the size of the device and the required skill for operating the device. Modern defibrillators are designed for recognising a shockable rhythm and clearly distinguishing a shockable rhythm from a non-shockable one. Most such devices are further equipped with prompts or displays giving clear and unambiguous instructions for a layperson to observe when assisting as a first-responder a person under cardiac arrest. At the same time, the defibrillators also provide for an advanced, i. e. manual, mode for skilled emergency personnel, such as fire-fighters, EMT's and physicians.

One problem arising from the use of defibrillators is that so far, shocks which are sufficiently strong to defibrillate the heart may also cause temporary or even permanent damage to the heart. The event of receiving a defibrillation shock is accompanied by physical and structural trauma to the tissue of the patient. This is often a direct consequence of the energy delivered.

Damaged or scarred myocardial tissue is often a source for re-entry, a condition which causes fibrillation of the heart due to an electrical wavefront following a circular path in the heart instead the normal path of physiological excitation.

US 7,340,301 B1 describes a portable defibrillation system. The document discloses a procedure for controlling the power that will be transferred through an output transformer. A circuit enables a time delay from the crossing of the sine wave. A wave generator is disclosed for shaping the pulses within the limits of the sine wave with variable peak outputs and thus enabling the delivery of the exact amount of energy required. Still, this document remains silent on how to optimize the amount of energy delivered to a patient, such as to inflict a sufficiently reliable defibrillating shock with a minimum of disruptive energy. Cardiac cells form a functional network that allows rapid propagation of the electrical impulse and subsequent coordinated mechanical contraction. This process of excitation is dependent on the electrical activity of the cardiac cells (action potential), electrical cell-to-cell coupling and the overall architecture of the cellular network. The effect of pulses that are generated extra-cellularly on cardiac tissue is a complex one and depends on a plurality of factors, such as the timing during the cardiac cycle, the field strength of the shock, the underlying cardiac tissue architecture and the electrical properties of the cardiac cells.

It is therefore an object of the present invention to provide a defibrillator which overcomes the drawbacks of the prior art, which in particular provides a reliable and effective defibrillation shock, while at the same time reducing tissue and neuromuscular trauma for the patient. It is a further object of the present invention to provide a defibrillator with a decreased defibrillation threshold that can be produced in a cost-efficient manner as a comparatively smaller, low weight defibrillator and which uses little energy, for public and private use.

This has been solved with a defibrillator for delivering a defibrillation shock to the heart of a patient under cardiac arrest according to the independent patent claims. This defibrillator can be a manually operated defibrillator, e. g. a defibrillator that requires input from a skilled operator such as a physician or EMT and whose defibrillation shock is delivered upon an input from said operator, or an automated external defibrillator, that can be used by a wide range of operators and incorporate means for assisting lay or less skilled operators by performing analysis, giving output and/or automatically determining the best treatment. The defibrillator further comprises at least one energy source for providing electrical energy. Typically, defibrillators are equipped with at least one single use or rechargeable battery for providing the electrical energy needed for delivering the defibrillation shock. The defibrillator can also be equipped with a plurality of batteries and/or be directly plugged to the electrical grid. In particular, auxiliary batteries can be provided for supplying the continuous operating functions of the defibrillators with energy, such as for, for example, the periodic status controls and/or the analysis means. The defibrillator is further equipped with at least one capacitor for building up and storing an electrical charge for delivery to the patient. In a preferred embodiment, capacitors of from 5 µF up to 500 µF are used, more preferably capacitors from 50 µF to 200 µF, more preferably from 60 µF to 200 µF.

A defibrillator according to the present invention can provide a defibrillation shock with significantly reduced energy, sufficient to defibrillate the heart, while at the same time reducing stress and risk of injury to the myocardial tissue of a patient. The defibrillator thus further helps at preventing reentry.

The defibrillator is further provided with at least one set of electrodes to be applied externally to the patient's skin. A typical set of electrodes consists of at least two electrodes for conducting the electricity through the patient's thorax, but further electrodes such as for grounding, impedance measurement or for shaping an electrical field are also conceivable. The defibrillator according to the present invention is adapted to provide at least one pulse train.

Said pulse train consist of one individual pulse or is a sequence of individual pulses and comprises at least one individual pulse. The individual pulses have a duration from 0.4 to 3 ms, preferably of from 0.5 to 2 ms, even more preferably of from 1 to 2 ms.

In a further preferred embodiment, the individual pulses are of a duration of 0.5 ms, alternatively 1 ms, further alternatively 1.5 ms and further alternatively 2 ms. In an alternative embodiment of the present invention, the pulse train consists of at least two individual pulses.

In the context of the present invention, a pulse is to be understood as a transitional change from a base line value to a higher or lower value as a function of time. A pulse train according to the present invention is to be understood as a sequence of individual pulses. The time interval from the end of a first pulse up to the starting of an eventual second pulse is defined as interpulse duration. In the course of the present invention, the base line refers to the voltage if not expressively stated otherwise. The change in the voltage baseline is essentially reflected in the change in transmembrane potential caused by the single pulse, therefore a change in the voltage causes a corresponding change in the transmembrane potential of the myocardial cells. Individual pulses according to the present invention are therefore further defined as causing a change in the transmembrane potential of from zero to 100%, each individual pulse in the pulse train causing the transmembrane potential to start from zero again.

In a preferred embodiment, the pulse train comprises pulses of variable, preferably alternating, polarities. In a further preferred embodiment the pulse train consists of a series of two individual pulses, whereby a first pulse has a first polarity and the second pulse has an opposite polarity in respect to the first.

In a preferred embodiment, the capacitor is a high voltage capacitor. Such a high voltage capacitor would be adapted for high voltages in the range of 2000 to 4000 V.

In a further preferred embodiment, a series of capacitors is employed. Such capacitors can be the above capacitors adapted for voltages in the range of 2000 V to 4000 V. In a particular embodiment of the present invention, each individual pulse is provided by the discharge of one capacitor, i.e. the device comprises a number of capacitors, each adapted to provide an individual pulse in a pulse train.

In a further preferred embodiment, the individual pulses of one pulse train have a duration of less than 2 ms.

In a further preferred embodiment, the individual pulses of a pulse train are further chopped into subpulses. These subpulses can have a duration of from about 5% to about 50% of the total duration of the pulse. In a further preferred embodiment, the subpulses have a duration of from about 0.1 to 0.5 ms. The chopping of pulses into subpulses according to the present invention shall mean, that the individual pulses are themselves separated into subpulses by inactivity delays within the pulse. In further preferred embodiments, the subpulses have a duration of 0.3 ms. An individual pulse can be chopped into a series of subpulses by having a plurality of inactivity delays. In a preferred embodiment, a subpulse is chopped by at least one inactivity delay, preferably one to three inactivity delays. The inactivity delays may have a duration in respect to the overall duration of the pulse of from about 5 to about 50% of the total pulse duration. Preferably, the inactivity delays have a duration of from 0.05 to 0.5 ms, even more preferably 0.1 ms.

According to the present invention, a subpulse is defined with respect to the change in transmembrane potential caused. The subpulses differ from the pulses, in that during an inactivity delay, the change in transmembrane potential does not drop below a certain threshold, preferably the change in transmembrane potential does not drop for more than 50%, more preferably for not more than 75%. In a most preferred embodiment, the change in transmembrane potential does not drop to zero in an inactivity delay between subpulses. By contrast, the change in transmembrane potential reaches essentially zero during the interpulse duration between the pulses. Each individual subpulse causes a change of transmembrane potential from an elevated potential, preferably the subpulses cause a change in transmembrane potential of above 0%, more preferably of above 20%, even more preferably of more than 50%.

The subpulses can be generated by switching a capacitor on and off at the desired frequency. Preferably, the number of capacitors corresponds to the number of subpulses within a single pulse. Instead of switching a single capacitor on/off while delivering subpulses, each capacitor is fully discharged when the pulse is generated in a sequence corresponding to the subpulse frequency.

It has been found that the efficient energy delivered to the cardiac cells can be reduced by interrupting the delivered pulses by one or several short intervals, i.e. inactivity delays, while at the same time minimally affecting the maximal level of transmembrane potential of the cardiac cells.

It has surprisingly been found, that the envelope of the change in transmembrane potential was minimally affected by the above interruptions of the shortened pulse and reached the maximal level of transmembrane potential as if the pulse would not have been interrupted. For a decrease of 13 % of total pulse time, no statistically significant decrease in transmembrane potential has been detected.

In a particular embodiment, the subpulses can be of variable, preferably alternating polarities. A first pulse of a positive polarity can be followed by a second subpulse of negative polarity, which in term can be followed by a third pulse of again positive polarity, for instance.

In a particular embodiment, each consecutive subpulse after a first subpulse is of a sequentially inverted polarity in respect to the precedent subpulse.

In a preferred embodiment of the present invention, the individual pulses have an essentially square waveform. Alternatively, the pulses have an essentially linear or essentially exponential decreasing waveform.

In a further preferred embodiment, the pulse train comprises at least two pulses with an interpulse duration of from 0.5 to 500 ms, preferably 0.5 to 30 ms, preferably less than 30 ms, even more preferably with an interpulse duration of 1 ms.

In a further preferred embodiment, the pulse train consists of two pulses with an interpulse duration of from 0.5 to 500 ms, preferably 0.5 to 30 ms, preferably less than 30 ms, even more preferably with an interpulse duration of 1 ms.

In a further preferred embodiment, the defibrillator further comprises means for measuring patient impedance. Such means for measuring patient impedance are well known in the art and can be derived from e. g. WO03/055557(A1).

In a further preferred embodiment, the present invention provides a defibrillator with further means for adapting the pulses to compensate for higher or lower than average patient impedance. It is known in the art that patient impedance can vary considerably. For obese patients, for instance, the impedance is differs from slim patients, while for infants and for paediatric use, for instance, the impedance is lower due to a reduced chest volume. It is paramount to ensure that each patient receives a defibrillation pulse sufficient to defibrillate the heart and low enough such as to avoid undue trauma and damage to the tissue. Therefore, the strength of the pulse has to be adapted for the varying degrees of patient impedance. Typically, the amount of energy provided is expressed as joules per kg. A system for adapting the pulse to patient impedance can be adapted from e. g. WO03/055557(A1). In the context of the present invention, the impedance adjustment of the defibrillation pulse is preferably performed by adjusting the pulse amplitude, as opposed to pulse duration.

In a further preferred embodiment, the defibrillator according to the invention further comprises means for analysing an ECG and in the case of automated external defibrillators means for determining the presence and/or absence of a shockable rhythm. Such means are known in the art and can be derived from e. g. US 2006/0025825 or US 6,287,328. A further preferred embodiment also comprises means for analysing an ECG while artefacts are present, such for example as artefacts resulting from cardiopulmonary resuscitation. An example for an artefact compensated ECG analysis system is shown in EP 2 172 245 A1.

In a preferred embodiment, the defibrillator is adapted to provide pulse(s) with a total energy per pulse train of less than 200 joules; preferably the defibrillator is adapted to provide pulses with a total energy of less than 130 joules, even more preferably of less than 60 joules.

In a further preferred embodiment, the defibrillator is adapted to provide pulses of a duration lower than the time constant of a myocardial tissue, preferably lower than 3.6 ms.

A further aspect of the present invention relates to a method of operating an external defibrillator. An energy source is provided, a capacitor is provided, and electrodes are provided. Energy is then provided from the energy source to the capacitor and at least one pulse train is discharged from the capacitor. Such a pulse train comprises and/or consists of at least one individual pulse of a duration of from 0.4 to 3 ms, preferably of a duration of from 0.5 to 2 ms, even more preferably of from 1 to 2 ms.

In a preferred embodiment, the individual pulses of a pulse train are provided with variable, preferably alternating polarities.

In a further preferred embodiment, a high voltage capacitor is provided.

In a further preferred embodiment several capacitors are provided.

In a further preferred embodiment, the pulses are further chopped into subpulses, each of a duration of from about 5% to about 50% of the total duration of the individual pulse. Preferably said duration is of from about 0.1 to 0.5 ms. By chopping the pulses into subpulses inactivity delays are generated between individual subpulses. Said inactivity delays can last from about 5% to about 50% of the total duration of the pulse. Preferably, the inactivity delays last from 0.05 to 0.5 ms, even more preferably they last 0.1 ms. Preferably, a capacitor is provided for each subpulse to be delivered. Each capacitor is fully charged and all the capacitors are discharged in consecutive order in a frequency determined by the desired inactivity delays.

By means of the present invention it becomes possible to reduce the delivered energy of a defibrillation pulse, while at the same time supplying sufficient energy to defibrillate the heart and prevent re-entry or refibrillation of the heart. With a defibrillator according to the present invention, different causes of cardiac arrest such as ventricular fibrillation or ventricular tachycardia can be efficiently stopped while at the same time preventing undue trauma and damage to the patient's tissue. As the present defibrillator also requires a lower overall energy for producing a defibrillation shock, the size of the battery - one of the most important drivers for size and weight of defibrillators - can further be reduced and a simple, easy to manufacture and storable defibrillator be provided.

The invention will be further outlined in the following in reference to the preferred embodiments with examples and drawings without being limited thereto.
Fig. 1: shows state of the art defibrillator waveforms usable in the present invention in
   a) damped sine wave monophasic,
   b) truncated exponential biphasic
   c) rectilinear biphasic and
   d) triphasic
Fig. 2: shows a defibrillation pulse train according to the present invention with
   a) single pulse,
   b) dual biphasic pulse
Fig. 3: shows pulse trains with chopped single pulses according to the present invention with
   a) inactivity delays,
   b) inactivity delays and biphasic,
   c) inactivity delays and alternating polarities of the subpulses, and
   d) inactivity delays and alternating polarities of the subpulses and biphasic
Fig. 4: shows the variation in transmembrane potential (ΔVₘ) induced by single pulses with inactivity delays of
   a) 0.1 ms
   b) 0.2 ms
   c) 0.5 ms
Fig. 5: shows a schematic standard defibrillator set-up according to the invention
Fig. 6: shows a graphic of the energy for defibrillation thresholds in function of time

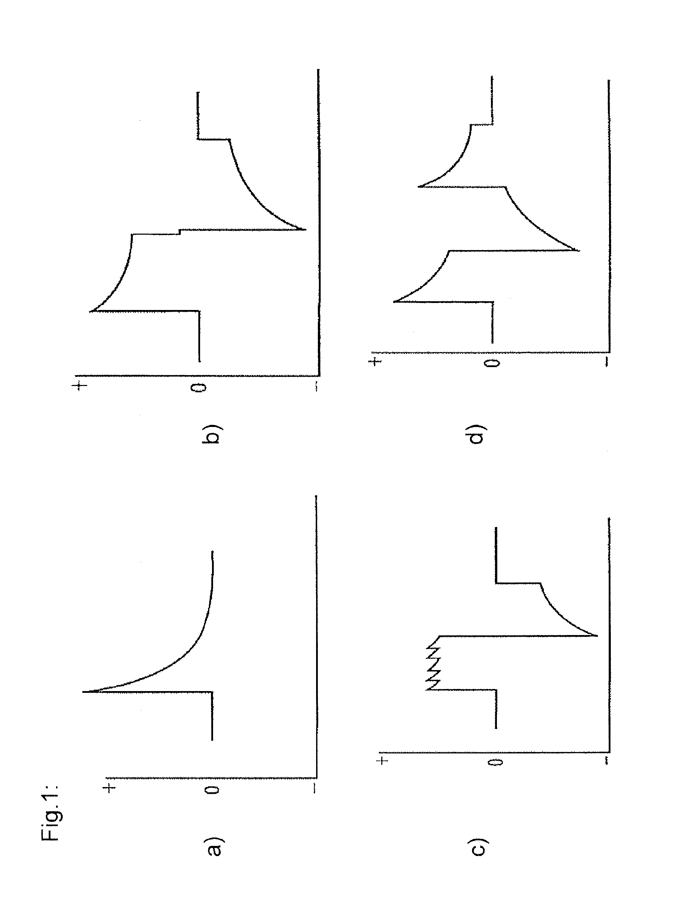

Figure 1 gives an overview of frequently used defibrillator waveforms which are known in the art. Figure 1 a) shows a damped sine wave monophasic waveform. Figure 1 b) shows a truncated exponential biphasic waveform. Figure 1 c) shows a rectilinear biphasic waveform, and figure 1 d) depicts a triphasic waveform.

All these waveform can potentially be used according to the present invention by shortening the pulse duration. The duration of the individual pulses is reduced from the frequently used 3.5 ms to 5 ms to a duration of from 0.5 to 2 ms. Furthermore, the interpulse duration is also reduced.

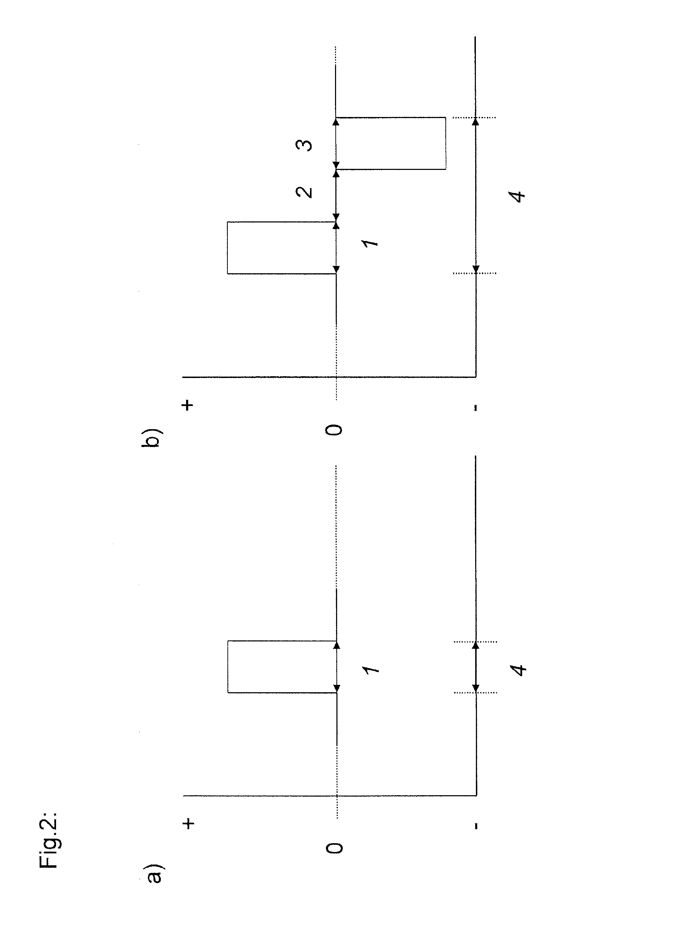

Figures 2a and b shows waveforms according to a preferred embodiment of the present invention. The waveforms are essentially square waveforms. Figure 2 a) shows a monophasic single-pulse pulse train 4 with a duration 1 of 1.5 ms. Figure 2 b) shows a pulse train 4 consisting of two individual pulses. A first pulse has a first pulse duration 1 of 1 ms followed by an interpulse duration 2 of 1 ms and a successive pulse of inverted polarity with a second duration 3 of 1 ms.

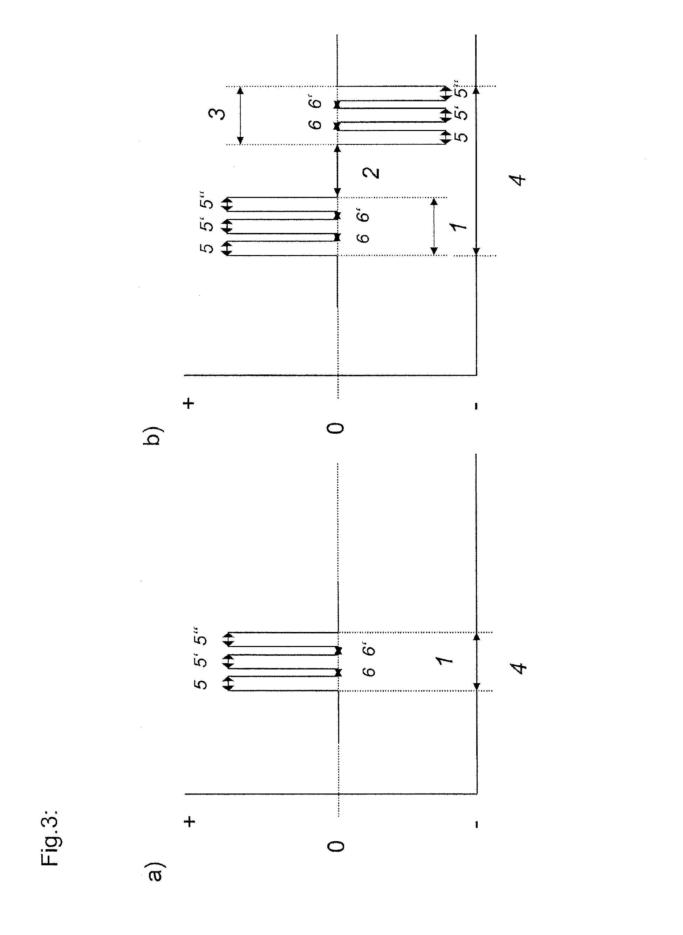

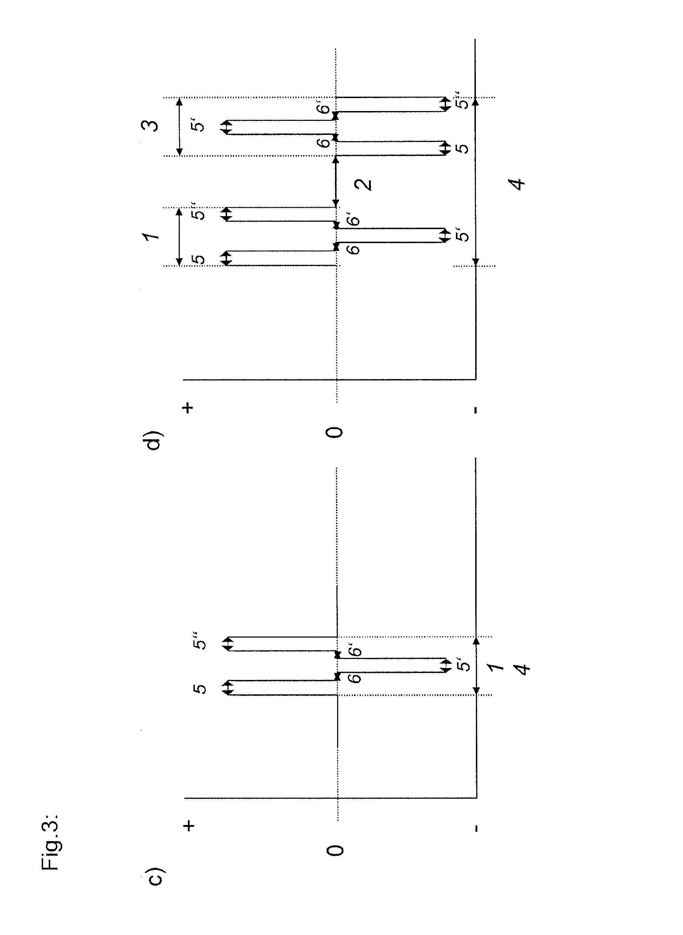

Figures 3a to d shows further pulses according to the present invention. Figure 3 a) shows a single-pulse pulse train 4 with a total duration 1 of the single pulse of 1.1 ms. The single pulse is chopped into three subpulses by two inactivity delays 6 and 6'. The first inactivity delay 6 has a duration of 0.1 ms, the second inactivity delay 6' has a duration of 0.1 ms. The subpulses have a duration 5, 5', 5" of 0.3 ms each. Figure 3 b) shows a pulse train 4 consisting of two individual pulses, whereby the second pulse is of an opposite polarity to the first. The first individual pulse has a first duration 1 of 2 ms. The interpulse duration 2 is 1 ms and the second pulse has a second pulse duration 3 of 2 ms. The first pulse is chopped into three subpulses, each with a duration 5, 5' and 5" of 0.6 ms and separated by inactivity delays 6 and 6' of 0.1 ms. The second pulse is equally chopped into three subpulses with durations 5, 5' and 5" of 0.6 ms, separated by inactivity delays 6 and 6' of 0.1 ms.

Figure 3 c) shows an exemplary pulse train 4 consisting of a single pulse with a duration 1 of 2 ms. The individual pulse is further chopped into three subpulses, each separated from the precedent subpulse by an inactivity delay. The first inactivity delay 6 lasts for 0.1 ms, and the second inactivity delay 6' lasts for 0.1 ms. The first subpulse has a first duration 5 of 0.6 ms and is followed by the inactivity delay 6 of 0.1 ms. The second subpulse is of an inverted polarity in respect to the first subpulse and has a duration 5' of 0.6 ms. The second subpulse is in turn followed by an inactivity delay 6' of 0.1 ms followed by a third subpulse with a third duration 5" of 0.6 ms. Figure 3 d) shows a pulse train 4 consisting of two individual pulses which in turn are chopped into three individual subpulses each. The first subpulse has a duration 1 of 1.1 ms and the second individual pulse has a second duration 3 of 1.1 ms. The individual pulses are separated by an interpulse duration 2 of 1 ms. The subpulses have durations 5, 5' and 5" of 0.1 ms and inactivity delays 6 and 6 of 0.1 ms. Each of the subpulses is followed by a subpulse of opposite polarity.

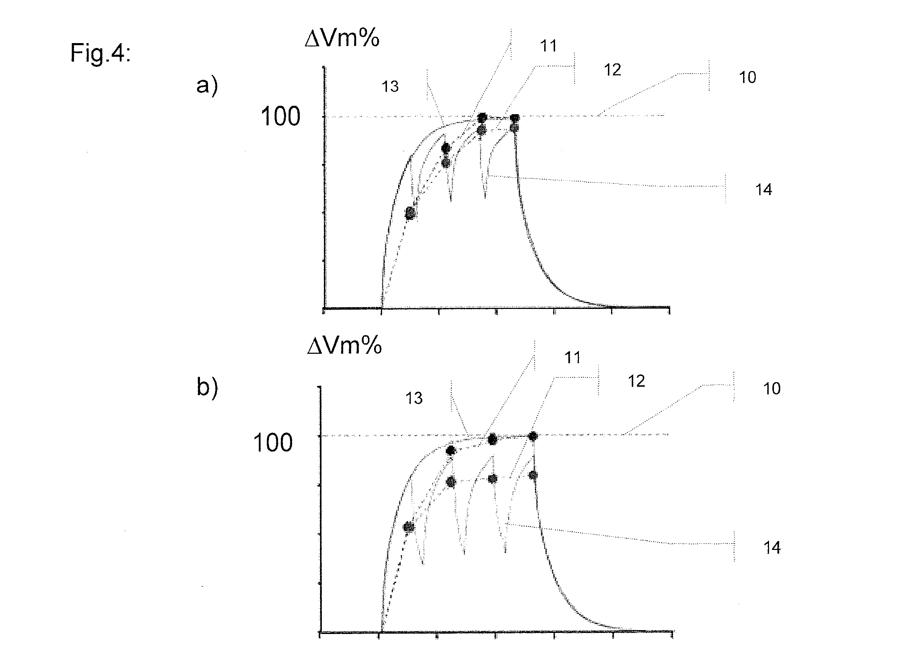

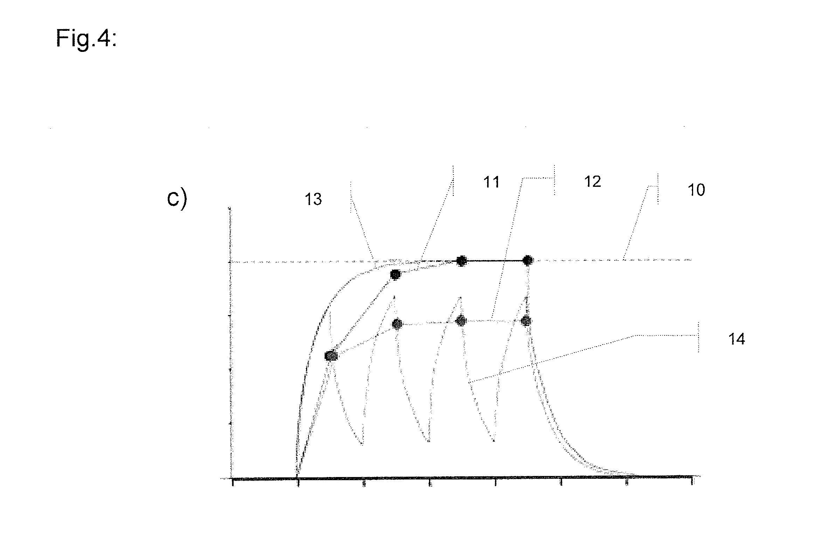

An electrical pulse on the heart causes changes in the transmembrane action potential (ΔVₘ) by initiating new action potentials or prolonging local refractory periods and thereby contributing to the interruption of fibrillatory activity. Figure 4 shows ΔVₘ recorded at the border of tissue induced by single pulses according to the present invention. The upper traces 11 correspond to a continuous pulse, whereas the lower traces 12 correspond to pulses with inactivity delays.

Patterned cell cultures from neonatal rat heart cells were used for this experiment. The heart from neonatal rats was excised, enzymatically digested to form a cell suspension and preplated three times for elimination of fibroblasts and seeded on cover-slips at a density of of 0.5 x 10³ cells per ml. Before seeding, a coat of fibronectin forming defined patterns was produced by microphotolithography to determine cell attachment. In such a way, defined geometrical cell culture boundaries could be produced. After seeding, the cell cultures were kept in an incubator for six to eight days at a temperature of 35° C. For determination of ΔVₘ linear strands were patterned with a strand width of 200 µm.

The graphs 4 a) to 4 c) show, that it was possible to reduce the energy of the delivered pulse by interrupting a square pulse by at least one or more very short inactivity delays. The threshold of 100% ΔVₘ. 10 is the asymptotic line reached by transmembrane potential. 13 is the modelled ΔVₘ change for a single pulse of 2.3 ms duration. 14 is the modelled ΔVₘ change for a chopped pulse of 4 subpulses of a duration of 0.5 ms by three interval delays of duration 0.1 ms. 11 is the measured ΔVₘ change on the cell culture for a single pulse of 2.3 ms duration. 12 is the measured ΔVₘ change on the cell culture for chopped pulses of 4 subpulses of duration 0.5 ms by 3 interval delays of 0.1 ms duration.

It has been shown that the inactivity delays lower the level of the maximally reached ΔVₘ but do not affect the general time course of the voltage change. Surprisingly, the envelope of the change in ΔVₘ was not affected by the inactivity delays, which reached a maximal level of membrane potential at essentially the same time and value.

Figures 4a to 4c also serve at distinguishing the material difference between a pulse and a subpulse. While the figures represent a single pulse each, it can be seen that the transmembrane potential is essentially the same (typically zero) before and after discharge of the pulse. The subpulses on the other hand are timed with their inactivity delays such that the transmembrane potential never drops below a certain threshold, e.g. does not reach zero in the particular embodiment depicted in Figures4a to 4c.

In essence, any defibrillator can be adapted by the person skilled in the art for exercising the present invention. For example defibrillators "FRED easy" by Schiller Medical S.A. (F-67162 Wissembourg) can be adapted by the person skilled in the art for exercising the present invention.

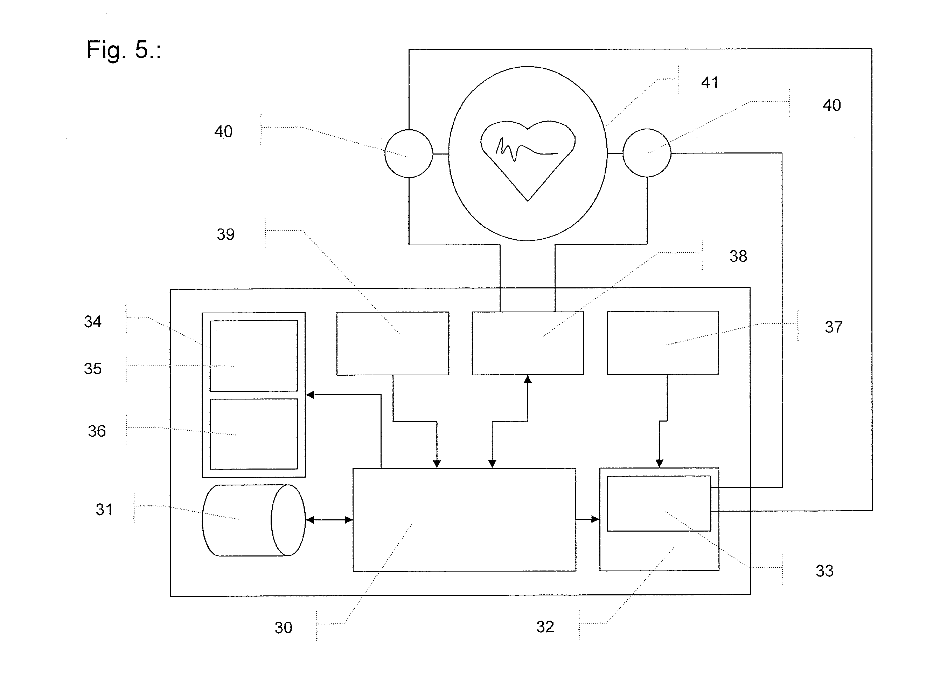

Figure 5 shows an exemplary defibrillator or AED set up that can be adapted for exercising the present invention. An AED generally comprises a battery 37 supplying energy to a capacitor 32. A wave shaper 33 can be used to form a desired wave shape, such as for example an essentially square waveform. An output unit 34 is further included, comprising an optical output monitor 35 and a voice prompt 36. The defibrillator further includes an ECG sensing circuit 38. Detection elements such as electrode pads 40 are attached thereto. The user can interact with the machine through a user interface 39. During operation, a processor 30 is responsible for controlling the various components according to the protocol and user input. The processor 30 is further capable of storing data or retrieving data from a data storage unit 31. During operation, the electrode pads 40 are attached to a patient's thorax 41 and function as sensors and shock delivery elements at the same time.

The advantages conferred by the present invention shall be further conferred by means of examples and comparison with the prior art.

### Example I:

Table I shows the efficiency of defibrillation pulses in an animal model. Different Voltages (V) were applied and the rate of successful defibrillation recorded in %.

Four durations were tested with pulse trains consisting of one individual pulse. The pulses had durations of 0.5 ms, 1 ms, 1.5 ms and 2 ms. All pulses were rectangular. A two third (66.6 %) or more efficiency of defibrillation was marked as a successful defibrillation. As can be seen in Table I efficiency reaches an optimum at 1.5 ms with adequate results with lowered Voltage at 2ms. With pulses of a duration of more that 2 ms the efficiency reaches a plateau with no further improvements.

**Table I: Efficiency of Single Pulses**

| **Efficiency** | | | | |
|---|---|---|---|---|
| **v** | 0, 5 ms | 1 ms | 1, 5 ms | 2 ms |
| 3000 | 0% | **100%** | n/a | n/a |
| 2500 | n/a | 57,10% | **100%** | 50% |
| 2000 | n/a | 60% | **100%** | **100%** |
| 1500 | n/a | 33,30% | **66,70%** | **80%** |
| 1000 | n/a | 0% | 25% | 0% |

### Example II:

Table II shows the efficiency of defibrillation pulse trains with two individual pulses in an animal model. Different Voltages (V) were applied and the rate of successful defibrillation recorded in %. The pulses were tested with the same polarity in the successive pulse (+/+) and with alternating polarity in the second pulse (+/-). Tests were performed with decreasing voltages in steps of 500 V beginning with 2500 V.

One duration was tested with individual pulses in the pulse trains of a duration of 1 ms with an interpulse duration of 1 ms. Efficiency shows an optimum at lowest voltage for biphasic pulses of a duration of 1ms.

**Table II: Efficiency of Double Pulses**

| **Efficiency** | | |
|---|---|---|
| | 1 ms / 1 ms | |
| V | +/+ | +/- |
| 2500 | **100%** | n/a |
| 2000 | **100%** | **100%** |
| 1500 | **100%** | **100%** |
| 1000 | 0% | **67%** |

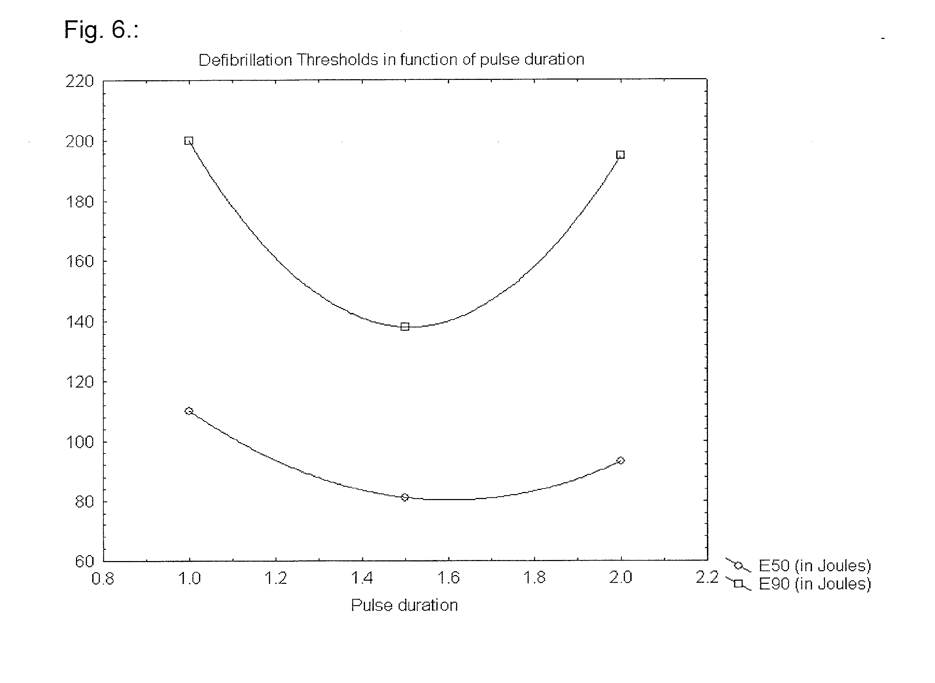

Figure 6 shows the energy needed to successfully defibrillate in an animal model (defibrillation threshold) in function of the duration of the pulse in ms. It has surprisingly been found, that efficiency is greater at about 1.5 ms (lowest defibrillation threshold). E50 and E90 are the defibrillation thresholds providing 50 %, respectively 90 % probability of shock efficiency.

## Claims

1. An external defibrillator for delivering a defibrillation shock to the heart of a patient under cardiac arrest, comprising
a) at least one energy source for providing electrical energy;
b) at least one capacitor for building up and storing an electrical charge for delivery to the patient;
c) at least one set of electrodes to be applied externally to the patient's skin;
**characterized in that**,
the external defibrillator is adapted to provide at least one **pulse train** comprising at least one **individual pulse** of a duration of from 0.4 to 3 ms, preferably of from 0.5 to 2 ms, even more preferably of from 1 to 2 ms.

2. The external defibrillator of claim 1, whereby the at least one pulse train comprises pulses of **variable,** preferably **alternating,** polarities.

3. The external defibrillator of claim 1 or 2, whereby the at least one capacitor is a **high voltage capacitor.**

4. The external defibrillator of any one of claims 1 to 3, whereby the at least one individual pulse(s) have a duration of less than 2 ms.

5. The external defibrillator of any one of claims 1 to 4, whereby the at least one individual pulse(s) are further **chopped** into **subpulses** of a duration of from about 5% to about 50% of the total duration of the pulse, preferably of from 0.1 to 0.5 ms, thereby generating **inactivity delays** between the individual subpulses, said inactivity delays lasting from about 5% to about 50% of the total duration of the pulse, preferably from 0.05 to 0.5 ms.

6. The external defibrillator of claim 5, whereby the at least one pulse(s) comprises subpulses of **variable,** preferably **alternating,** polarities.

7. The external defibrillator of claim 5 or 6, whereby each **consecutive subpulse** after the first subpulse is of a **sequentially inverted,** preferably alternating, polarity in respect to the precedent subpulse.

8. The external defibrillator of any one of claims 1 to 7, whereby the **waveshape** of the at least one pulse(s) is an essentially **square wave, essentially linear wave or essentially exponential wave.**

9. The external defibrillator of any one of claims 1 to 9, whereby the at least one pulse train consists of one, or comprises at least two, pulses with an **interpulse duration** of from 0.5 to 30 ms, preferably 1 ms.

10. The external defibrillator of any one of claims 1 to 9, whereby the defibrillator further comprises **means for measuring patient impedance** between the electrodes and preferably further **means for adjusting the pulses to compensate** for higher or lower than average patient impedance.

11. The external defibrillator of any one of claims 1 to 10, whereby the defibrillator further comprises **means for analyzing an ECG** and **determining** based on said analyzing the presence of a shockable rhythm.

12. The defibrillator of any one of claims 1 to 11, whereby the defibrillator is adapted to provide pulse(s) with a total energy of less than 200 J, preferably of less than 130 J.

13. The defibrillator of any one of claims 1 to 12, whereby the defibrillator is adapted to provide pulses of a duration **lower than a time constant of a myocardial tissue.**

14. A method of operating an automated external defibrillator comprising the steps of:
a) providing at least one energy source;
b) providing at least one capacitor;]
c) providing electrodes;
**characterized in that** a an energy from the energy source is provided to the capacitor and at **least one pulse train** comprising **at least one individual pulse** of a duration of from 0.4 to 3 ms, preferably of from 0.5 to 2 ms, even more preferably of from 1 to 2 ms is discharged from the at least one capacitor.

15. The method according to claim 14, wherein the pulses are further **chopped** into **subpulses** of a duration of from about 5% to about 50% of the total duration of the pulse, preferably of from 0.1 to 0.5 ms, thereby generating **inactivity delays** between the individual subpulses, said inactivity delays lasting from about 5% to about 50% of the total duration of the pulse, preferably from 0.05 to 0.5 ms.
